# DEMANDE DE BREVET EUROPEEN

(11) **EP 1 702 608 A1**
(43) Date de publication de la demande: **20.09.2006**
(21) Numéro de dépôt: 06110293.5
(22) Date de dépôt: 22.02.2006
(51) Int. Cl.: A61K 8/365, A61K 8/39, A61K 8/60, A61Q 19/10, A61Q 5/02, A61Q 5/12

(54) **Composition cosmétique de nettoyage contenant un composé d'urée et un composé oxyalkyléné**

(30) Priorité: 17.03.2005 FR 0550685
(71) Demandeur: L'Oreal-D.I.P.I., 75008 Paris (FR)
(72) Inventeur: Aubrun-Sonneville, Odile, 92160, Antony (FR)
(74) Mandataire: Rasson, Catherine

(57) **Abrégé**

L'invention se rapporte à une composition cosmétique de nettoyage contenant dans un milieu aqueux, (1) au moins un composé oxyalkyléné non ionique (2) au moins un composé de formule (I) suivante : 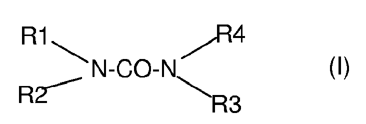 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères, et (3) au moins un tensioactif moussant.

Le composé de formule (I) est de préférence l'hydroxyéthylurée.

L'invention se rapporte aussi à l'utilisation de la dite composition comme produit de nettoyage de la peau, produit de démaquillage de la peau et/ou des lèvres, produit de gommage de la peau ou produit de nettoyage des cheveux.

## Description

L'invention se rapporte à une composition cosmétique de nettoyage contenant dans un milieu aqueux, un composé d'urée hydroxylé, un composé oxyalkyléné non ionique et un tensioactif moussant, et à ses utilisations pour le nettoyage et/ou le démaquillage de la peau humaine, des lèvres, et/ou des cheveux.

Le nettoyage de la peau est très important pour le soin du visage, et il doit être le plus performant possible car les résidus gras tels que l'excès de sébum, les restes des produits cosmétiques utilisés quotidiennement et les produits de maquillage s'accumulent dans les replis cutanés et peuvent obstruer les pores de la peau et entraîner l'apparition de boutons.

Pour nettoyer la peau, il est connu d'utiliser des gels moussants. Ces produits sont des solutions aqueuses de tensioactifs moussants, utilisées pour leurs propriétés détergentes, et généralement épaissies par des polymères, afin d'en faciliter la manipulation. Les dérivés oxyalkylénés non ioniques sont couramment utilisés pour épaissir les gels moussants car ils conduisent à des produits transparents dont la qualité de mousse reste bonne.

Toutefois, ces produits présentent souvent l'inconvénient de manquer de confort à l'application en laissant une sensation de tiraillements et de sécheresse. En effet, une des difficultés majeures de la formulation de produits moussants consiste à mettre au point des produits ayant un très bon pouvoir moussant pour assurer un bon nettoyage et pouvant se rincer facilement, tout en respectant la barrière cutanée, c'est-à-dire sans dessécher la peau.

Un moyen d'améliorer la sensation de confort et d'hydratation est d'ajouter des agents hydratants comme la glycérine. Cependant, ce type de dérivé a tendance à fluidifier les compositions moussantes les contenant, ce qui entraîne une modification de la texture et peut altérer les qualités des compositions qui deviennent plus difficiles à manipuler.

Il subsiste donc le besoin de disposer de compositions cosmétiques de nettoyage, qui ne soient pas desséchantes pour la peau et qui aient néanmoins une texture adaptée à l'utilisation envisagée et notamment une viscosité satisfaisante.

La demanderesse a découvert de manière surprenante qu'il est possible d'obtenir des produits nettoyants, apportant un effet hydratant, sans compromettre la texture de la composition, en introduisant au moins un dérivé d'urée dans des gels moussants épaissis par un composé oxyéthyléné non ionique. Le gel moussant contient un ou plusieurs tensioactifs moussants.

Ainsi, l'invention a pour objet une composition cosmétique de nettoyage contenant dans un milieu aqueux, (1) au moins un composé oxyalkyléné non ionique, (2) au moins un composé de formule (I) suivante : 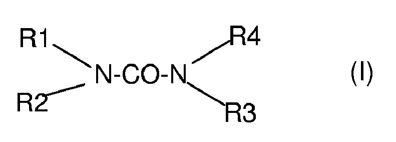 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères, et (3) au moins un tensioactif moussant.

La composition de l'invention présente l'avantage de ne pas dessécher la peau tout en ayant une viscosité satisfaisante. L'utilisation du composé de formule (I) comme hydratant permet de manière inattendue de conserver le pouvoir épaississant des composés oxyéthylénés, ce qui n'est pas possible avec d'autres hydratants comme la glycérine. Aucun document ne suggère que les composés de formule (I) permettent de conserver le pouvoir gélifiant des composés oxyéthylénés non ioniques dans des moussants.

Dans la présente demande, on entend par « milieu aqueux », un milieu contenant de l'eau et éventuellement un ou plusieurs solvants organiques hydrosolubles. Dans ce milieu aqueux, la quantité d'eau est de préférence d'au moins 20 % en poids ; elle va de préférence de 20 à 95 % en poids, mieux de 30 à 90 % en poids et encore mieux de 40 à 85 % en poids par rapport au poids total de la composition.

En outre, la composition de l'invention étant une composition cosmétique, elle contient un milieu physiologiquement acceptable. On entend ici par « milieu physiologiquement acceptable », un milieu compatible avec la peau, les muqueuses, les yeux. II s'agit de préférence d'un milieu cosmétiquement acceptable, c'est-à-dire qui, en outre, présente une couleur, une odeur et un toucher agréables et qui ne génère pas d'inconforts inacceptables (picotements, tiraillements, rougeurs), susceptibles de détourner la consommatrice d'utiliser cette composition.

Les compositions selon l'invention peuvent se présenter sous forme de solutions épaissies, de gels, de laits ou de crèmes plus ou moins épaisses, et elles peuvent s'écouler ou non sous leur propre poids selon leur viscosité. La viscosité mesurée à 25°C avec l'appareil de mesure Rheomat 180 à 200 rpm (tours par minute) peut aller par exemple de 0,5 à 100 Pa.s (5 à 1000 poises), plus particulièrement entre 0,8 et 50 Pa.s. Le Rheomat 180 est équipé d'un mobile différent selon les viscosités, par exemple d'un mobile 3 pour la gamme des viscosités de 0,2 à 4 Pa.s, et d'un mobile 4 pour la gamme des viscosités supérieures à 2 Pa.s. Cette viscosité est mesurée généralement 10 minutes après la mise en rotation du mobile.

De façon avantageuse, le pH du milieu aqueux est compatible avec les matières kératiniques et notamment avec la peau. Ce pH va de préférence de 3 à 8,5, mieux de 3,5 à 8, préférentiellement de 5 à 7,5.

La composition de nettoyage de l'invention constitue une composition moussante qui est rincée après application sur la peau.

### Composé d'urée hydroxylé

Le composé d'urée présent dans la composition selon l'invention est un composé de formule (I) suivante : 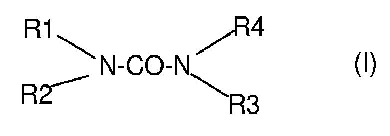 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères.

Dans les composés de formule (I) :
- De préférence R1 désigne un groupe hydroxyalkyle en C2-C6 et R2, R3, R4 désignent, indépendamment l'un de l'autre, un atome d'hydrogène ou un groupe alkyle en C1-C4 ;
- Préférentiellement R1 désigne un groupe hydroxyalkyle en C2-C6 comprenant de 1 à 5 groupes hydroxyles, notamment 1 groupe hydroxyle, et R2, R3, R4 désignent un atome d'hydrogène ;
- Plus préférentiellement, R1 désigne un groupe hydroxyalkyle en C2-C4 comprenant 1 groupe hydroxyle et R2, R3, R4 désignent un atome d'hydrogène.

Comme groupes alkyle en C1-C4, on peut citer notamment les groupes méthyle, éthyle, n-propyle, isopropyle, n-butyle, iso-butyle et tert-butyle.

Parmi les groupes hydroxyalkyle en C1-C6, on préfère ceux contenant un seul groupe hydroxyle et en particulier les groupes hydroxyéthyle, hydroxypropyle, hydroxybutyle, hydroxypentyle et hydroxyhexyle.

Comme sels de tels composés, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, acide sulfonique ou acide phosphonique. II peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (I) préférés, on peut citer le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée ; le N-méthyl-N-(1,3,4,5,6-pentahydroxy-2-hexyl)-urée ; le N-méthyl-N'-(1-hydroxy-2-méthyl-2-propyl)-urée ; le N-(1-hydroxy-2-méthyl-2-propyl)-urée ; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée; le N,N-Bis-(2-hydroxyéthyl)-N',N'-dimethyl-urée ; le N,N,N',N'-tetrakis-(2-hydroxyéthyl)-urée ; le N',N'-Bis-(2-hydroxyéthyl)-N',N'-bis-(2-hydroxypropyl)-urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société National Starch sous la dénomination commerciale Hydrovance® .

Le composé de formule (I) peut être présent dans la composition selon l'invention en une quantité (en matière active) allant de 0,1 % à 50 % en poids, par rapport au poids total de la composition, de préférence allant de 0,1 % à 20 % en poids, et préférentiellement allant de 0,1 % à 10 % en poids.

### Composés oxyalkylénés

La composition selon l'invention comprend un ou plusieurs composés oxyalkylénés non ioniques qui sont des composés comprenant au moins un groupe choisi parmi les groupes d'oxyde d'éthylène (OE), les groupes d'oxyde de propylène (OP) et leurs mélanges (OE OP). Les composés peuvent donc être des composés oxyéthylénés, des composés oxypropylénés, ou des composés oxyéthylénés / oxypropylénés. Ces composés ne sont pas des tensioactifs mais ils ont des propriétés épaississantes.

La quantité de composé(s) oxyalkyléné(s) non ionique(s) dans la composition de l'invention, en poids de matière active, peut aller par exemple de 0,1 à 15 % en poids, mieux de 0,2 à 10 % en poids et encore mieux de 0,2 à 5 % en poids par rapport au poids total de la composition.

Les composés oxyalkylénés non ioniques peuvent être choisis notamment parmi les polyéthylène glycols, les esters d'acide gras et de polyéthylène glycol et/ou de polypropylène glycol, les éthers d'alcool gras et de polyéthylène glycol et/ou de polypropylène glycol, les dérivés alkyl- ou acyl-alkoxylés et notamment de polyol, les triesters de glycérol et d'acides gras oxyalkylénés et notamment oxyéthylénés, les dérivés d'amides gras oxyéthylénés ou oxypropylénés, les dérivés uréthanes oxyéthylénés modifiés par des chaînes alkyle, et leurs mélanges.
1. Les polyéthylène glycols pouvant être utilisés dans la composition de l'invention sont des polycondensats d'oxyde d'éthylène. De préférence, ces polyéthylène glycols ont un nombre de motifs d'oxyde d'éthylène (OE) supérieur 1000. Le nombre d'oxyde d'éthylène peut aller par exemple de 1000 à 50000 et de préférence de 5000 à 10000. Comme polyéthylène glycols, on peut citer par exemple le polyéthylène glycol comportant 7000 OE (nom CTFA : PEG-7M) comme le produit commercialisé sous la dénomination POLYOX WSR N-750® par la société Amerchol, le polyéthylène glycol comportant 14000 OE (nom CTFA : PEG-14M) comme le produit commercialisé sous la dénomination POLYOX WSR 205 par la société Amerchol, le polyéthylène glycol comportant 20000 OE (nom CTFA : PEG-20M) comme le produit commercialisé sous la dénomination POLYOX WSR 1105® par la société Amerchol.
2. Les esters d'acide gras et de polyéthylène glycol et/ou de polypropylène glycol sont des condensats de polyéthylène glycol et/ou polypropylène glycol avec un ou plusieurs acides gras. Ce sont des composés de formule (II) :

   RCOO - (OE)m - (OP)n - R' (II)

   dans laquelle 0<m<300 et 0<n<300 et m+n > 6, R et R' représentent indépendamment l'un de l'autre l'hydrogène ou une chaîne alkyle saturée ou insaturée, linéaire ou ramifiée, hydroxylée ou non, comportant de 1 à 30 atomes de carbone et de préférence de 12 à 22 atomes de carbone, ou une chaîne aryle, à condition que R et R' ne soit pas en même temps l'hydrogène.
   Comme esters d'acide gras et de polyéthylène glycol et/ou de polypropylène glycol, on peut citer par exemple le distéarate de polyéthylène glycol (150 OE) tel que le produit commercialisé sous la dénomination ATLAS G-1821® par la société Uniqema, le PEG-150 dibehenate tel que le produit commercialisé sous la dénomination ETHOX PEG 6000 Dibehenate® par la société Ethox, le palmito-stéarate de polyéthylène glycol (120 OE) tel que le produit commercialisé sous la dénomination STEARATE 6000 WL 1644® par la société Gattefosse, le copolymère de polyéthylène glycol (30 OE) et d'acide 12- hydroxystéarate tel que le produit commercialisé sous la dénomination ARLACEL P135® par la société Uniqema, le stéarate de polyéthylène glycol (40 OE) tel que le produit commercialisé sous la dénomination MYRJ 52® par la société Uniqema.
   Dans le cas où dans la formule (II), R=R'=H, on peut citer par exemple le copolymère statistique polyoxyéthylène / polyoxypropylène (17 OE / 6 OP) commercialisé sous la référence UCON 75-H-450® par la Société Amerchol. Les molécules comportant plus d'OE et/ou plus d'OP ne sont pas exclues.
3. Les éthers d'alcool gras de polyéthylène glycol et/ou de polypropylène glycol sont des condensats de polyéthylène glycol et/ou polypropylène glycol avec un ou plusieurs alcools gras. Ce sont des composés de formule (III) :

   R - (OE)m - (OP)n - R' (III)

   dans laquelle 0<m<300 et 0<n<300 et m+n > 6, R et R' représentent indépendamment l'un de l'autre l'hydrogène ou une chaîne alkyle saturée ou insaturée, linéaire ou ramifiée, hydroxylée ou non, comportant de 1 à 30 atomes de carbone et de préférence de 12 à 22 atomes de carbone, ou une chaîne aryle, à condition que R et R' ne soit pas en même temps l'hydrogène.
   Comme exemples d'éthers d'alcool gras et de polyéthylène glycol, on peut citer notamment l'alcool laurylique oxyéthyléné (4 OE) tel que le produit commercialisé sous la dénomination BRIJ 30® par la société Uniqema, l'alcool cétylique oxyéthyléné (30 OE) tel que le produit commercialisé sous la dénomination NIKKOL BC-30TX® par la société Nikkol, l'alcool oléylique oxyéthyléné (15 OE) tel que le produit commercialisé sous la dénomination NIKKOL BO-15TX® par la société Nikkol, l'alcool oléylique oxyéthyléné (50 OE) tel que le produit commercialisé sous la dénomination NIKKOL BO-50® par la société Nikkol, l'alcool béhénylique oxyéthyléné (10 OE) tel que le produit commercialisé sous la dénomination MERGITAL B 10® par la société Nikkol, l'alcool béhénylique oxyéthyléné (30 OE) tel que le produit commercialisé sous la dénomination NIKKOL BB-30® par la société Nikkol, l'alcool laurylique oxyéthyléné (12 OE) tel que le produit commercialisé sous la dénomination REWOPAL 12® par la société Goldschmidt, l'alcool laurylique oxyéthyléné (23 OE) tel que le produit commercialisé sous la dénomination SIMULSOL P 23® par la société Seppic, l'alcool octyl-2 dodécylique oxyéthyléné (20 OE) tel que le produit commercialisé sous la dénomination OCTYLDODECETH-20® par la société Stearinerie Dubois, l'alcool iso-cétylique oxyéthyléné (20 OE) tel que le produit commercialisé sous la dénomination ARLASOLVE 200 US® par la société Uniqema, l'alcool oléylique oxyéthyléné (10 OE) tel que le produit commercialisé sous la dénomination BRIJ 97® par la société Uniqema, l'alcool oléylique oxyéthyléné (20 OE) tel que le produit commercialisé sous la dénomination BRIJ 98® par la société Uniqema, l'alcool stéarylique oxyéthyléné (100 OE) tel que le produit commercialisé sous la dénomination BRIJ 700® par la société Uniqema, l'alcool stéarylique oxyéthyléné (21 OE) tel que le produit commercialisé sous la dénomination BRIJ 721® par la société Uniqema.
   Comme exemple d'éther de polypropylène glycol, on peut citer notamment l'alcool myristylique oxypropyléné (3 OP) tel que le produit commercialisé sous la dénomination PROMYRISTYL PM-3® par la société Croda,
   Comme exemples d'éthers de polyéthylène glycol/polypropylène glycol, on peut citer notamment l'alcool laurylique oxyéthyléné (5 OE) oxypropyléné (5 OP) tel que le produit commercialisé sous la dénomination AETHOXAL B® par la société Cognis, l'alcool cétylique oxyéthyléné (20 OE) oxypropyléné (5 OP) tel que le produit commercialisé sous la dénomination PROCETYL AWS® par la société Croda, l'alcool butylique oxyéthyléné (26 OE) oxypropyléné (26 OP) tel que le produit commercialisé sous la dénomination PPG-26-BUTETH-26® par la société Goldschmidt, l'alcool butylique oxyéthyléné (26 OE) oxypropyléné (26 OP) tel que le produit commercialisé sous la dénomination VARONIC APEB®) par la société Goldschmidt, le décyl-tetra-decanol oxyéthyléné (30 OE) oxypropyléné (6 OP) tel que le produit commercialisé sous la dénomination NIKKOL PEN-4630® par la société Nikkol, l'alcool laurylique oxyéthyléné (25 OE) oxypropyléné (25 OP) tel que le produit commercialisé sous la dénomination ADF-OLEILE®) par la société Vevy.
4. Les dérivés alkyl ou acyl alkoxylés de polyol peuvent être notamment des dérivés alkyl ou acyl éthoxylés de polyol, par exemple des dérivés oxyéthylénés d'esters d'acide gras et de polyol ou des dérivés oxyéthylénés d'éthers d'alcool gras et de polyol, et notamment des dérivés oxyéthylénés d'esters d'acide gras ou d'éthers d'alcool gras et de glycérol ou de sorbitol ou de glucose ou de pentaérythritol.
   Comme dérivés de ce type, on peut citer par exemple, le cocoate de glycéryle oxyéthyléné (78 OE) tel que le produit commercialisé sous la dénomination SIMULSOL CG par la société Seppic, le di-oléate de méthyl-glucose oxyéthyléné (120 OE) (nom CTFA : PEG-120 méthylglucose dioléate) tel que le produit commercialisé sous la dénomination GLUCAMATE DOE-120 VEGETAL® par la société Amerchol, le septa-oléate de sorbitane oxyéthyléné (40 OE) tel que le produit commercialisé sous la dénomination ARLATONE T ® par la société Uniqema, le laurate de polyglycéryle (2 moles de glycérol) oxyéthyléné (10 OE) tel que le produit commercialisé sous la dénomination HOE S 3495 ® par la société Clariant, l'isostéarate de glycéryle oxyéthyléné (60 OE) tel que le produit commercialisé sous la dénomination EMALEX GWIS-160 ® par la société SACI-CFPA, le monostéarate de glycéryle oxyéthyléné (20 OE) tel que le produit commercialisé sous la dénomination CUTINA E 24 ® par la société Cognis, le stéarate de glycéryle oxyéthyléné (200 OE) tel que le produit commercialisé sous la dénomination SIMULSOL 220 TM ® par la société Seppic, le tétrastéarate de pentaérythrityle oxyéthyléné (150 OE) tel que le produit commercialisé sous la dénomination CROTHIX ® par la société Croda, le tristérate de sorbitan oxyéthyléné (160 OE) tel que le produit commercialisé sous la dénomination RHEODOL TW IS399C par la société.Kao Chemicals.
5. Comme triesters de glycérol et d'acides gras oxyalkylénés, on peut citer par exemple les glycérides d'acide caprylique/caprique oxyéthylénés (6 OE), tels que le produit commercialisé sous la dénomination SOFTIGEN 767® par la société Condea, et l'huile d'olive oxyéthylénée (50 OE) tel que le produit commercialisé sous la dénomination CROVOL O-70® par la société Croda.
6. Comme dérivés d'amides d'acide gras oxyalkylénés, on peut citer notamment les amides d'acide gras oxypropylénés comme par exemple le PPG-2 HYDROXYETHYL COCAMIDE et les mélanges en contenant tels que les produits commercialisés par Uniqema sous la dénomination Promidium, notamment Promidium CO.
7. Comme dérivés uréthanes oxyéthylénés modifiés par des chaînes alkyle, on peut citer par exemple ceux de formules (III) et (IV) :

   R₁ NH-CO-(OCH₂CH₂) ₐ-[O-CO-NR₄-R₃-NR₄-CO-(OCH₂CH₂) ₐ]_{b}-O-CO-NH R₂ (III)

   R₅-(OCH₂CH₂) ₙ-O-CO-NH-R₆-NH-CO-(OCH₂CH₂) ₙ-OR₅ (IV)

   dans lesquelles les radicaux R₁, R₂ et R₅ représentent un groupe alkyle de C₁₋₁₈ ; R₃ et R₆ représentent un radical hydrocarboné linéaire, cyclique ou aromatique en C₄₋₃₆ ; R₄ représente un atome d'hydrogène ou un radical alkyle en C₁₋₆, de préférence un atome d'hydrogène ; a et n sont des nombres entiers allant de 90 à 600, et b est un nombre entier allant de 1 à 4.

Il s'agit par exemple de polymères hydrosolubles obtenus par réaction d'addition de diisocyanates (HMDI : Hexamethylene diisocyanate) sur des diols (polyéthers ou polyesters) et terminés par des groupements hydrophobes provenant d'alcools gras éthoxylés ou éthoxylés/propoxylés. C'est le cas par exemple du NUVIS FX 1100, commercialisé par la société Elementis, qui est un copolymère alcool stéarylique oxyéthyléné (100 OE) / polyéthylène glycol (136 OE) /hexaméthylène diisocyanate (nom CTFA : steareth-100/PEG-136/HMDI copolymer).

Selon un mode préféré de réalisation de l'invention, le composé oxyalkyléné non ionique est choisi parmi les polyéthylène glycols, les dérivés oxyéthylénés d'esters ou d'éthers d'acide gras et de polyol, notamment avec ou sans méthylglucose ou pentaérythritol, les dérivés d'amides d'acide gras oxyéthylénés ou oxypropylénés, les dérivés uréthanes oxyéthylénés modifiés par des chaînes alkyle, et leurs mélanges.

### Tensioactif moussant

La composition moussante selon l'invention contient au moins un tensioactif moussant qui va apporter le caractère moussant à la composition. Ce tensioactif peut être choisi parmi les tensioactifs moussants non ioniques, anioniques, amphotères et zwitterioniques et leurs mélanges.

Les tensioactifs moussants sont des détergents et se différencient des émulsionnants par la valeur de leur HLB (Hydrophilic Lipophilic balance), le HLB étant le rapport entre la partie hydrophile et la partie lipophile dans la molécule. Le terme HLB est bien connu de l'homme du métier et est décrit par exemple dans "The HLB system. A time-saving guide to Emulsifier Selection" (published by ICI Americas Inc ; 1984). Pour les émulsionnants, le HLB va généralement de 3 à 8 pour la préparation des émulsions E/H et de 8 à 18 pour la préparation des émulsions H/E, alors que les tensioactifs moussants ont généralement un HLB supérieur à 20.

La quantité de tensioactif(s) moussants peut aller par exemple, en poids de matière active, de 1 à 50 % en poids et mieux de 2 à 20 % en poids par rapport au poids total de la composition.

### 1. Les tensioactifs non ioniques :

Les tensioactifs non ioniques peuvent être choisis par exemple parmi les alkyl polyglucosides (APG), les esters de maltose, les alcools gras polyglycérolés, les dérivés de glucamine comme l'éthyl-2 hexyl oxy-carbonyl n-méthyl glucamine, et leurs mélanges.

Comme alkylpolyglucosides, on utilise de préférence ceux contenant un groupe alkyle comportant de 6 à 30 atomes de carbone et de préférence de 8 à 16 atomes de carbone, et contenant un groupe hydrophile (glucoside) comprenant de préférence 1,2 à 3 unités de glucoside. Comme alkylpolyglucosides, on peut citer par exemple le decylglucoside (Alkyl-C9/C11-polyglucoside (1.4)) comme le produit commercialisé sous la dénomination MYDOL 10® par la société Kao Chemicals, le produit commercialisé sous la dénomination PLANTAREN 2000 UP® par la société Cognis, et le produit commercialisé sous la dénomination ORAMIX NS 10 ® par la société Seppic ; le caprylyl/capryl glucoside comme le produit commercialisé sous la dénomination ORAMIX CG 110® par la Société Seppic ; le laurylglucoside comme les produits commercialisés sous les dénominations PLANTAREN 1200 N® et PLANTACARE 1200® par la société Cognis ; le coco-glucoside comme le produit commercialisé sous la dénomination PLANTACARE 818/UP® par la société Cognis ; et leurs mélanges.

Les dérivés de maltose sont par exemple ceux décrits dans le document EP-A-566438, tels que l'O-octanoyl-6'-D-maltose, ou encore le O-dodecanoyl-6'-D-maltose décrit dans le document FR-2,739,556.

Parmi les alcools gras polyglycérolés on peut citer le dodecanediol polyglycérolé (3,5 moles de glycérol), produit commercialisé sous la dénomination CHIMEXANE NF® par la société Chimex.

2. Les tensioactifs anioniques peuvent être choisis par exemple parmi les savons (sels alcalins d'acides gras), les carboxylates, les acylaminoacides, les amidoéther carboxylates, les alkyl polyaminocarboxylates, les alkyl éthers sulfates tels que les Sodium Laureth sulfates, les alkyl sulfonates, les iséthionates, les alkyl méthyltaurate, les alkyl sulfosuccinates, les alkyl sulfoacétates, les alkylphosphates (mono ou dialkylphosphates), leurs sels, et leurs mélanges.

Comme carboxylates, on peut citer notamment les acides alkyl glycol carboxyliques (ou acides 2-(2-Hydroxyalkyloxy acétique), et leurs sels tels que par exemple le lauryl glycol carboxylate de sodium, commercialisé sous les dénominations BEAULIGHT SHAA® ou BEAULIGHT LCA-25N® par la société SANYO (nom CTFA : Sodium Lauryl Glycol Carboxylate), ou sa forme acide correspondante commercialisée sous la dénomination BEAULIGHT SHAA (Acid Form)® par la société SANYO.

Comme acylaminoacides, on peut citer par exemple le cocoylglycinate de sodium commercialisé par la société Ajinomoto sous la dénomination Amilite GCS 12.

Comme alkylphosphates, on peut citer par exemple le laurylphosphate commercialisé par la société Kao sous la dénomination MAP 20.

3. Les tensioactifs moussants amphotères et zwitterioniques peuvent être choisis par exemple parmi les dérivés de bétaïnes dont les amidopropylbétaïnes, les amphoacétates et les amphodiacétates, les hydroxylsultaines et leurs mélanges.

Comme dérivés de bétaïnes, on peut citer par exemple la cocobétaïne comme le produit commercialisé sous la dénomination DEHYTON AB-30® par la société Cognis ; la laurylbétaïne comme le produit commercialisé sous la dénomination GENAGEN KB® par la société Clariant ; la laurylbétaïne oxyethylénée (10 OE), comme le produit commercialisé sous la dénomination LAURYLETHER(10 OE)BETAINE® par la société Shin Nihon Rica ; la stéarylbétaïne oxyéthylénée (10 OE) comme le produit commercialisé sous la dénomination STEARYLETHER(10 OE)BETAINE® par la société Shin Nihon Rica ; la cocamidopropyl betaine commercialisé par exemple sous la dénomination VELVETEX BK 35® par la société Cognis ; le undecylenamidopropyl betaïne commercialisé par exemple sous la dénomination AMPHORAM U® par la société Ceca ; et leurs mélanges.

Comme amphoacétates et amphodiacétates, on peut citer par exemple le N-cocoyl-N-carboxyméthoxyéthyl-N-carboxyméthyl-éthylènediamine N-di-sodique (nom CTFA: disodium cocamphodiacetate) comme le produit commercialisé sous la dénomination MIRANOL C2M CONCENTRE NP® par la société Rhodia Chimie ; le N-cocoyl-N-hydroxyéthyl-N-carboxyméthyl-éthylènediamine N-sodique (nom CTFA : sodium cocamphoacetate) et leurs mélanges.

Au moins un des tensioactifs moussants est de préférence choisi parmi les alkylpolyglucosides, les dérivés de bétaïne, les acides alkyl glycol carboxyliques et leurs sels, les alkyl éthers sulfates, les alkylphosphates, les amphodiacétates, les amphoacétates, les alkylglycinates et leurs mélanges.

### Adjuvants

Comme indiqué ci-dessus, le milieu aqueux de la composition selon l'invention peut contenir, outre l'eau, un ou plusieurs solvants hydrosolubles choisis parmi les alcools inférieurs (mono-alcools) comportant de 1 à 6 atomes de carbone, tels que l'éthanol ; et les polyols tels que la glycérine, les glycols comme le butylène glycol, l'isoprène glycol, le propylène glycol, les polyéthylène glycols tels que le PEG-8 ; le sorbitol ;les sucres tels que le glucose, le fructose, le maltose, le lactose, le sucrose ; et leurs mélanges, dans la mesure où ces composés n'altèrent pas les propriété recherchées de la composition selon l'invention. La quantité de solvant(s) dans la composition de l'invention peut aller par exemple de 0,1 à 30 % en poids, de préférence de 0,5 à 20 % en poids, mieux de 0,5 à 15 % en poids et encore mieux de 0,5 à 10 % en poids par rapport au poids total de la composition.

Les compositions de l'invention peuvent contenir aussi des adjuvants choisis parmi ceux habituellement utilisés dans le domaine cosmétique. Comme adjuvants, on peut citer par exemple les parfums, les conservateurs, les séquestrants (EDTA), les pigments, les charges notamment exfoliantes, les colorants solubles, les filtres solaires, les actifs cosmétiques ou dermatologiques tels que les hydratants comme l'acide hyaluronique ; les céramides ; les vitamines hydrosolubles ou liposolubles comme la vitamine C et ses dérivés tels que la vitamine CG ; les antiseptiques ; les antiséborrhéïques ; les antimicrobiens tels que le peroxyde de benzoyle, l'acide salicylique, le triclosan, l'acide azélaïque ; les azurants optiques. Les quantités de ces différents adjuvants sont celles classiquement utilisées dans le domaine considéré, et par exemple de 0,01 à 5 % du poids total de la composition. Ces adjuvants ainsi que leurs concentrations doivent être tels qu'ils ne modifient pas la propriété recherchée pour la composition de l'invention.

Comme exfoliants, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Ces particules peuvent être présentes en une quantité allant par exemple de 0,5 à 30 % en poids, de préférence de 1 à 20 % en poids et mieux de 1 à 10 % en poids par rapport au poids total de la composition. Quand la composition contient des particules exfoliantes, elle peut constituer notamment une composition de gommage de la peau du visage ou du corps.

Les compositions de l'invention peuvent contenir aussi des adjuvants habituellement utilisés dans le domaine des nettoyants moussants comme les polymères. Bien que ces polymères puissent être non ioniques, ils sont de préférence choisis parmi les polymères anioniques, les polymères cationiques et leurs mélanges.

Comme polymères cationiques ou amphotères, on peut citer notamment ceux du type Polyquaternium (nom CTFA), qui apportent douceur et onctuosité à la crème moussante. Ces polymères peuvent être choisis de préférence parmi les polymères suivants :
- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA ;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP ;
- Polyquaternium 39 tel que le produit MERQUAT PLUS 3330 commercialisé par la société CALGON ;
- Polyquaternium 44 tel que le produit LUVIQUAT CARE commercialisé par la société BASF ;
- Polyquaternium 46 tel que le produit LUVIQUAT HOLD commercialisé par la société BASF ;
- Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

Comme polymères anioniques, on peut citer notamment ceux comportant au moins une chaîne hydrophobe, et en particulier ceux dérivés d'acide acrylique ou méthacrylique, comme le copolymère acrylates/steareth-20 methacrylate commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), en émulsion aqueuse commercialisé sous la dénomination Aculyn 28 par la société Rohm & Haas ; le copolymère acide acrylique/itaconate de mono-cétyle oxyéthyléné (20 OE), en dispersion aqueuse à 30 % commercialisé sous la dénomination Structure 3001 par la société National Starch ; le copolymère acide acrylique/itaconate de mono-stéaryle oxyéthyléné (20 OE) en dispersion aqueuse à 30 % commercialisé sous la dénomination Structure 2001 par la société National Starch ; le copolymère acrylates/acrylate modifié par des alcools en C12-C24 polyoxyéthylénés (25 OE), sous forme de latex à 30-32 % de copolymère, commercialisé sous la dénomination Synthalen W2000 par la société 3V SA, ainsi que les copolymères vendus sous les noms PEMULEN ou CARBOPOL par la Société Noveon, comme le copolymère acrylate/C₁₀-C₃₀-alkylacrylate tel que les produits PEMULEN TR1, PEMULEN TR2 ou CARBOPOL 1382 (nom CTFA : Acrylates/C10-30 Alkyl acrylate Crosspolymer).

Comme polymères anioniques, on peut citer notamment ceux comportant au moins une chaîne hydrophobe, et en particulier ceux dérivés d'acide acrylique ou méthacrylique, comme le copolymère acrylates/steareth-20 methacrylate commercialisé sous la dénomination Aculyn 22 par la société Rohm & Haas (nom CTFA : Acrylates/Steareth-30 Methacrylate copolymer) ; le terpolymère acide (méth)acrylique / acrylate d'éthyle / méthacrylate de béhényle oxyéthyléné (25 OE), en émulsion aqueuse commercialisé sous la dénomination Aculyn 28 par la société Rohm & Haas ; le copolymère acide acrylique/itaconate de mono-cétyle oxyéthyléné (20 OE), en dispersion aqueuse à 30 % commercialisé sous la dénomination Structure 3001 par la société National Starch ; le copolymère acide acrylique/itaconate de mono-stéaryle oxyéthyléné (20 OE) en dispersion aqueuse à 30 % commercialisé sous la dénomination Structure 2001 par la société National Starch ; le copolymère acrylates/acrylate modifié par des alcools en C12-C24 polyoxyéthylénés (25 OE), sous forme de latex à 30-32 % de copolymère, commercialisé sous la dénomination Synthalen W2000 par la société 3V SA, ainsi que les copolymères vendus sous les noms PEMULEN ou CARBOPOL par la Société Noveon, comme le copolymère acrylate/C₁₀-C₃₀-alkylacrylate tel que les produits PEMULEN TR1, PEMULEN TR2 ou CARBOPOL 1382 (nom CTFA : Acrylates/C10-30 Alkyl acrylate Crosspolymer).

La composition peut contenir aussi des polymères comportant au moins un monomère à groupement sulfonique, et notamment les polymères et copolymères d'acide 2-acrylamido-2-méthylpropane sulfonique (AMPS) tels que :
- l'homopolymère réticulé et neutralisé d'acide 2-acrylamido 2-méthylpropane sulfonique, commercialisé par la société Clariant sous la dénomination commerciale « Hostacerin AMPS » (nom CTFA : ammonium polyacryldimethyltauramide) ;
- les copolymères anioniques réticulés d'acrylamide ou méthylacrylamide et d'acide 2-acrylamido-2-méthylpropane sulfonique, notamment ceux se présentant sous la forme d'une émulsion E/H, tels que ceux commercialisés sous le nom de SEPIGEL 305 par la société Seppic (nom C.T.F.A. : Polyacrylamide / C13-14 Isoparaffin / Laureth-7), sous le nom de SIMULGEL 600 par la société Seppic (nom C.T.F.A. : Acrylamide / Sodium acryloyldimethyltaurate copolymer /Isohexadecane / Polysorbate 80),
- les copolymères d'acide(méth)acrylique ou de (méth)acrylate et d'acide 2-acrylamido- 2-méthylpropane sulfonique, notamment ceux se présentant sous la forme d'une émulsion E/H, tels que ceux commercialisés sous le nom de SIMULGEL NS par la société Seppic (copolymère d'acrylamido-2-methyl propane sulfonate de sodium / hydroxyethyl acrylate en émulsion inverse à 40% dans Polysorbate 60 et squalane) (nom CTFA : hydroxyethyl acrylate/sodium acryloyldimethyltaurate copolymer / squalane / polysorbate 60) ou ceux commercialisés sous le nom de SIMULGEL EG par la société Seppic (copolymère d'acide acrylique / acrylamido-2-methylpropane-sulfonique sous forme de sel de sodium en émulsion inverse à 45% dans isohexadecane / eau) (nom C.T.F.A. : Sodium Acrylate / Sodium acryloydimethyl-taurate copolymer / Isohexadecane /Polysorbate 80),
- les copolymères d'acide 2-acrylamido-2-méthylpropane sulfonique et de vinylpyrrolidone ou de vinylformamide, tels que les produits commercialisés sous les dénominations ARISTOFLEX AVC par la société Clariant,
- les polymères d'AMPS modifiés hydrophobes comme notamment le copolymère d'AMPS et de methacrylate d'alcool en C12-C14 éthoxylé (copolymère non réticulé obtenu à partir de Genapol LA-070 et d'AMPS) (nom CTFA : Ammonium Acryloyldimethyltaurate / Laureth-7 Methacrylate Copolymer) commercialisé sous la dénomination ARISTOFLEX LNC par la société Clariant, et le copolymère d'AMPS et de méthacrylate de stéaryle éthoxylé (25 EO) (copolymère réticulé par le trimethylolpropane triacrylate, obtenu à partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant.

Ces polymères peuvent être en une quantité (en matière active) allant par exemple de 0,05 à 10 % en poids et mieux de 0,1 à 5 % en poids par rapport au poids total de la composition.

Les compositions selon l'invention peuvent être utilisées sur toutes les matières kératiniques telles que la peau, le cuir chevelu, les cheveux, les cils, les sourcils, les ongles ou les muqueuses, notamment comme produit d'hygiène, par exemple comme produit de nettoyage de la peau, des muqueuses et/ou des cheveux, en particulier comme produit de nettoyage et/ou de démaquillage de la peau (du visage et/ou du corps), comme produit de douche (produit deux-en-un), comme shampoing et après-shampoing, comme produit de rasage, comme masque à rincer, comme produit exfoliant (appelé aussi desquamant ou désincrustant) aussi bien pour le visage que pour le corps ou pour les mains, après addition de particules exfoliantes.

L'invention a encore pour objet l'utilisation cosmétique de la composition telle que définie ci-dessus, comme produit de nettoyage et/ou de démaquillage de la peau, comme produit de douche, comme shampoing ou après-shampoing, comme produit de rasage, comme masque à rincer, comme produit exfoliant.

Un autre objet de l'invention est un procédé de nettoyage d'une matière kératinique, telle que la peau, du cuir chevelu, des cheveux, des cils, des sourcils, des ongles ou des muqueuses, caractérisé en ce qu'on applique sur la matière kératinique, une composition telle que définie ci-dessus et qu'on la rince.

La matière kératinique est de préférence la peau.

Les compositions selon l'invention peuvent être par exemple utilisées de la manière suivante :
1) quand les compositions selon l'invention constituent des produits de nettoyage doux pour le visage, elles peuvent être utilisées de la manière suivante :
   - on fait mousser le produit dans les mains avec de l'eau
   - on applique la mousse sur le visage
   - on nettoie le visage
   - on rince à l'eau
2) quand les compositions selon l'invention constituent des produits démaquillants, elles peuvent être utilisées comme indiqué ci-dessus mais elles peuvent aussi être appliquées à sec sur le visage, puis massées jusqu'à obtention d'un démaquillage satisfaisant, et rincées à l'eau, ou bien elles peuvent être appliquées au moyen d'un coton.
3) on peut les utiliser comme produit de douche deux-en-un, comme shampooing et/ou après shampooing, comme masque à rincer ou comme produit de rasage, de la manière habituelle d'utilisation de ces produits.

Les exemples qui suivent servent à illustrer l'invention sans toutefois présenter un caractère limitatif. Les quantités indiquées sont en % en poids sauf mention contraire et correspondent à la quantité de matière active. Les noms des composés utilisés sont indiqués en nom CTFA ou en nom chimique.

### Exemples

| Composition | Exemple 1 de l'invention | Exemple 2 de l'invention | Exemple comparatif |
|---|---|---|---|
| Sodium Lauryl Glycol Carboxylate (1) | 3,9 | 3,9 | 3,9 |
| Decylglucoside (2) | 3,25 | 3,25 | 3,25 |
| PEG-150 pentaérythrityl tétrastéarate (3) | 1 | 1 | 1 |
| PEG-120 méthylglucose dioléate (4) | 1 | 1 | 1 |
| PEG-14M (5) | 1 | 1 | 1 |
| N-(2-hydroxyéthyl)-urée (6) | 10 | 5 | - |
| Glycérine | - | 5 | 10 |
| Conservateurs | 0,5 | 0,5 | 0,5 |
| Eau | Qsp 100 | Qsp 100 | Qsp 100 |
| pH | 7 | 7 | 7 |
| Viscosité au Rhéomat 180 (mobile 3) à 200 tours/minute | 1,4 Pa.s | 1,14 Pa.s | 0,67 Pa.s |

| | | | |
|---|---|---|---|
| (1) Beaulight Shaa commercialisé par la société Sanyo | | | |
| (2) MYDOL 10® ; commercialisé par la société KAO (à 40% en matière active) | | | |
| (3) CROTHIX commercialisé par la société Croda. | | | |
| (4) GLUCAMATE DOE-120 VEGETAL commercialisé par la société Amerchol. | | | |
| (5) POLYOX WSR 205 commercialisé par la société Amerchol. | | | |
| (6) Hydrovance commercialisé par la société National Starch (à 50% en poids en solution aqueuse). | | | |

Mode opératoire : On prépare une solution aqueuse des tensioactifs et des conservateurs. On chauffe vers 70°C et on ajoute le dérivé oxyalkyléné (PEG-14M). On refroidit le mélange à 25°C puis on y incorpore les hydratants (hydroxyéthylurée et/ou glycérine). On ajuste de pH à 7 et on complète l'eau pour avoir 100%.

Les exemples présentés ci-dessus montrent que les compositions nettoyantes selon l'invention présentent une viscosité supérieure à celle de l'exemple comparatif qui contient une quantité de glycérine équivalente à celle de l'hydroxyéthylurée dans l'exemple 1 selon l'invention. L'invention permet donc d'obtenir des produits plus épais et donc plus faciles à utiliser, tout en ayant une bonne hydratation.

### Exemples 3 à 6

| | Exemple 3 | Exemple 4 | Exemple 5 | Exemple 6 |
|---|---|---|---|---|
| Sodium lauryl éther sulfate | 6.5 | - | 6.5 | - |
| Coco-bétaine (1) | 6.5 | 6.5 | - | - |
| Coco-Glucoside (2) | - | - | 6.5 | 9.75 |
| Potassium Cocoyl Glycinate (3) | - | 6.5 | - | - |
| Lauryl phosphate (4) | - | - | - | 3.25 |
| PEG-120 Méthylglucose Dioléate (5) | 1 | - | - | 1 |
| PEG-150 distéarate | - | 2 | - | - |
| Laureth-4 (6) | - | - | 2 | - |
| PPG2 Hydroxyéthyl cocamide (7) | 1 | - | - | - |
| Steareth-100/PEG-136/HMD copolymer (8) | - | - | - | 1 |
| Silice (9) | - | 5 | - | - |
| Polyquaternium-7 | 0.3 | - | - | - |
| Acide salicylique | - | 0.2 | - | - |
| KOH | - | - | - | qs pH7 |
| N-(2-hydroxyéthyl)-urée (10) | 10 | 2.5 | 7 | 10 |
| Acide citrique | Qs pH7 | Qs pH7 | Qs pH7 | - |
| DMDM Hydantoin | 0.2 | 0.2 | 0.2 | 0.2 |
| Sodium Méthylparaben | 0.3 | 0.3 | 0.3 | 0.3 |
| Eau | Qsp100 | Qsp100 | Qsp100 | Qsp100 |

| | | | | |
|---|---|---|---|---|
| (1) Dehyton AB 30® commercialisé par la société Cognis (contenant 30% en poids de matière active). | | | | |
| (2) MYDOL 10® ; commercialisé par la société Kao (à 40% en matière active) | | | | |
| (3) Amilite GCS 12 commercialisé par la société Ajinomoto (solution aqueuse à 30% de matière active) | | | | |
| (4) MAP 20 commercialisé par la société Kao. | | | | |
| (5) GLUCAMATE DOE-120 VEGETAL commercialisé par la société Amerchol. | | | | |
| (6) BRIJ 30 commercialisé par la société Uniqema. | | | | |
| (7) Promidium CO commercialisé par la société Uniqema. | | | | |
| (8) Nuvis FX 1100 commercialisé par la société Elementis. | | | | |
| (9) Aerosil 200 commercialisé par la société Degussa-Hüls | | | | |
| (10) Hydrovance commercialisé par la société National Starch (à 50% de matière active en solution aqueuse) | | | | |

On obtient des produits ayant une texture agréable, qui sont faciles à utiliser et donnent une bonne mousse tout en étant hydratants.

## Revendications

1. Composition de nettoyage contenant dans un milieu aqueux, (1) au moins un composé oxyalkyléné non ionique, (2) au moins un composé de formule (I) suivante : 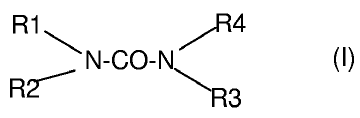 dans laquelle :
R1, R2, R3 et R4 représentent chacun, indépendamment l'un de l'autre, un atome d'hydrogène, un groupe alkyle en C1-C4 ou un groupe hydroxyalkyle en C2-C6 pouvant contenir de 1 à 5 groupes hydroxyles, où au moins un des radicaux R1 à R4 représente un groupe hydroxyalkyle, ainsi que leurs sels, leurs solvats, et leurs isomères, et (3) au moins un tensioactif moussant.

2. Composition selon la revendication 1, **caractérisée en ce qu'**elle comprend au moins 20 % en poids d'eau par rapport au poids total de la composition.

3. Composition selon la revendication 1 ou 2, **caractérisée en ce que**, dans les composés de formule (I), R1 désigne un groupe hydroxyalkyle en C2-C6 et R2, R3, R4 désignent, indépendamment l'un de l'autre, un atome d'hydrogène ou un groupe alkyle en C1-C4.

4. Composition selon l'une quelconque des revendications précédentes, **caractérisée en ce que**, dans les composés de formule (1), R1 désigne un groupe hydroxyalkyle en C2-C6 comprenant de 1 à 5 groupes hydroxyles, et R2, R3, R4 désignent un atome d'hydrogène.

5. Composition selon la revendication précédente, **caractérisée en ce que** R1 désigne un groupe hydroxyalkyle en C2-C6 comprenant 1 groupe hydroxyle.

6. Composition selon l'une quelconque des revendications précédentes, **caractérisée en ce que**, dans les composés de formule (I), R1 désigne un groupe hydroxyalkyle en C2-C4 comprenant 1 groupe hydroxyle et R2, R3, R4 désignent un atome d'hydrogène.

7. Composition selon l'une quelconque des revendications précédentes, **caractérisée en ce que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)-urée ; le N-(2,3,4,5,6-pentahydroxyhexyl)-urée; le N-méthyl-N-(1,3,4,5,6-pentahydroxy-2-hexyl)-urée; le N-méthyl-N'-(1-hydroxy-2-méthyl-2-propyl)-urée; le N-(1-hydroxy-2-méthyl-2-propyl)-urée; le N-(1,3-dihydroxy-2-propyl)-urée ; le N-(tris-hydroxyméthyl-méthyl)-urée ; le N-éthyl-N'-(2-hydroxyéthyl)-urée ; le N,N-bis-(2-hydroxyéthyl)-urée ; le N,N'-bis-(2-hydroxyéthyl)-urée ; le N,N-bis-(2-hydroxypropyl)-urée ; le N,N'-Bis-(2-hydroxypropyl)-urée ; le N,N-Bis-(2-hydroxyéthyl)-N'-propyl-urée ; le N,N-Bis-(2-hydroxypropyl)-N'-(2-hydroxyéthyl)-urée ; le N-tert.Butyl-N'-(2-(hydroxyéthyl)-N'-(2-(hydroxypropyl)-urée; le N-(1,3-dihydroxy-2-propyl)-N'-(2-hydroxyéthyl)-urée ; le N,N-Bis-(2-hydroxyéthyl)-N',N'-dimethyl-urée ; le N,N,N',N'-tetrakis-(2-hydroxy-éthyl)-urée ; le N',N'-Bis-(2-hydroxyéthyl)-N',N'-bis-(2-hydroxypropyl)-urée ; et leurs mélanges.

8. Composition selon l'une quelconque des revendications précédentes, **caractérisée en ce que** le composé de formule (I) est le N-(2-hydroxyéthyl)-urée.

9. Composition selon l'une quelconque des revendications précédentes, **caractérisée en ce que** les sels des composés de formule (I) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

10. Composition selon l'une quelconque des revendications précédentes, **caractérisée en ce que** le composé de formule (I) est présent en une quantité allant de 0,1 à 50 % en poids par rapport au poids total de la composition.

11. Composition selon l'une quelconque des revendications précédentes, **caractérisée en ce que** la quantité de composé(s) oxyalkyléné(s) non ionique(s) va de 1 à 10 % en poids par rapport au poids total de la composition.

12. Composition selon l'une quelconque des revendications précédentes, **caractérisée en ce que** le composé oxyalkyléné non ionique est choisi parmi les polyéthylène glycols, les esters d'acide gras et de polyéthylène glycol et/ou de polypropylène glycol, les éthers d'alcool gras et de polyéthylène glycol et/ou de polypropylène glycol, les dérivés alkyl ou acyl alkoxylés de polyol, les triesters de glycérol et d'acides gras oxyalkylénés, les dérivés d'amides gras oxyalkylénés, les dérivés uréthanes oxyéthylénés modifiés par des chaînes alkyle, et leurs mélanges.

13. Composition selon l'une quelconque des revendications précédentes, **caractérisée en ce que** le tensioactif moussant est choisi parmi les tensioactifs non ioniques, les tensioactifs anioniques, les tensioactifs amphotères et zwitterioniques et leurs mélanges.

14. Composition selon l'une quelconque des revendications précédentes, **caractérisée en ce que** au moins un tensioactif moussant est choisi parmi les alkylpolyglucosides, les dérivés de bétaïne, les acides alkyl glycol carboxyliques et leurs sels, les alkyl ethers sulfates, les alkylphosphates, les amphodiacétates, les amphoacétates, les alkylglycinates et leurs mélanges.

15. Composition selon l'une quelconque des revendications précédentes, **caractérisée en ce que** la quantité de tensioactif(s) moussant(s) va de 1 à 50 % en poids par rapport au poids total de la composition.

16. Composition selon l'une quelconque des revendications précédentes, **caractérisée en ce qu'**elle constitue un produit de nettoyage et/ou de démaquillage de la peau, un produit de douche, un shampoing, un après-shampoing, un produit de rasage, un masque à rincer, un produit exfoliant.

17. Utilisation cosmétique de la composition selon l'une quelconque des revendications 1 à 15, comme produit de nettoyage et/ou de démaquillage de la peau, comme produit de douche, comme shampoing, comme après-shampoing, comme produit de rasage, comme masque à rincer, comme produit exfoliant.

18. Procédé de nettoyage d'une matière kératinique, **caractérisé en ce qu'**on applique sur la matière kératinique, une composition selon l'une quelconque des revendications 1 à 15, et qu'on la rince.
